# EUROPÄISCHE PATENTSCHRIFT

(11) **EP 1 371 329 B1**
(45) Veröffentlichungstag und Bekanntmachung des Hinweises auf die Patenterteilung: **11.01.2012**
(21) Anmeldenummer: 03010642.1
(22) Anmeldetag: 13.05.2003
(51) Int. Cl.: A61B 5/15

(54) **Blutentnahmesystem**
Blood sampling system
Système de prélèvement du sang

(30) Priorität: 16.05.2002 DE 10222235
(43) Veröffentlichungstag der Anmeldung: 17.12.2003
(73) Patentinhaber: Roche Diagnostics GmbH, 68305 Mannheim (DE); F. Hoffmann-La Roche AG, 4070 Basel (CH)
(72) Erfinder: List, Hans, 64754 Hesseneck-Kailbach (DE); Ruschke, Peter, Dr., 55257 Budenheim (DE); VanHiel Brian, Smyrna, GA 30082 (US); Koeppel Bradley, Smyrna, GA 30080 (US); Kennedy Gwen, Ellenwood, GA 30294 (US)

(56) Entgegenhaltungen:
- EP-A- 1 034 740
- WO-A-93/09723
- US-A- 4 416 279
- US-A- 4 469 110
- US-A- 4 580 565
- US-A- 6 156 050

## Beschreibung

Die Erfindung betrifft ein Blutentnahmesystem zur Entnahme von Blut für Diagnosezwecke.

Als Blutentnahmesysteme werden im Sinne der Erfindung Lanzetten verwendet, die in ein entsprechendes Körperteil gestochen werden, um für analytische bzw. diagnostische Zwecke eine geringe Menge Blut aus einem Körperteil zu entnehmen. Häufig wird dabei das Blut aus dem Finger oder aus dem Ohrläppchen entnommen. In ärztlichen Praxen werden hierbei Lanzetten verwendet, die manuell oder mit einer einfachen Apparatur von dem Arzt oder dem Laborpersonal in das entsprechende Körperteil gestochen werden. Die Lanzette muss selbstverständlich scharf und steril sein. Im übrigen sind aber in der ärztlichen Praxis keine besonders hohen Anforderungen zu stellen, da die Blutentnahme bei einzelnen Patienten in großen zeitlichen Abständen durchgeführt werden und der Einstich durch trainiertes, speziell ausgebildetes Personal ausgeführt wird. Dennoch ist der Einstich häufig mit einem erheblichen Schmerz verbunden.

Soll das Blutentnahmesystem zur Handhabung durch den Patienten selbst geeignet sein, stellen sich wesentlich höhere Anforderungen an das System, die insbesondere eine schmerzarme und sichere Blutentnahme zum Ziel haben. Der eigenständige Gebrauch von Blutentnahmensystemen durch den Patienten erfolgt hierbei besonders im Rahmen des sogenannten Home-Monitoring-Bereiches. Hierbei soll besonders gefährdeten Patientengruppen eine regelmäßige Überwachung bestimmter analytischer Werte ihres Blutes ermöglicht werden. Dies gilt unter anderem für Diabetiker, die ihren Blutzuckerspiegel häufig und regelmäßig kontrollieren und diesen durch Insulininjektionen an den Bedarf anpassen. Der Bedarf an Insulin ist neben anderen Faktoren von der Nahrungsaufnahme und der körperlichen Aktivität abhängig und muss möglichst ständig innerhalb bestimmter Sollgrenzen gehalten werden. Dies ist für die Gesundheit der Patienten und die Vermeidung schwerwiegender Spätschäden, wie beispielsweise Erblindung und Amputation von Körperteilen von allergrößter Bedeutung.

Bereits seit langem werden Blutentnahmesysteme verwendet, die aus einem Stechgerät und zugehörigen, für das jeweilige Gerät speziell angepassten Lanzetten bestehen. In einem Gehäuse des Stechgerätes ist ein Lanzettenantrieb enthalten, durch den eine Lanzette mechanisch in die Haut eingestochen wird. Als Antriebselement für die Einstichbewegung dient in der Regel eine Feder. In der Anfangsphase der Entwicklung waren sehr einfache Konstruktionen gebräuchlich, bei denen die Lanzette unmittelbar an einem Ende einer im länglichen Gehäuse angeordneten Druckfeder befestigt war. Ein derartiges Blutentnahmegerät wird beispielsweise in dem Dokument US 4,469,110 offenbart. Bei dem Gebrauch der beschriebenen Systeme zeigt sich jedoch, dass insbesondere die Anforderung einer schmerzarmen Blutentnahme nicht erfüllt werden kann. Zur Reduktion des Einstichschmerzes wurden in neuerer Zeit kleine, einfach zu bedienende, verhältnismäßig kostengünstige Analysesysteme entwickelt, die meist zusätzlich aus Blutteststreifen und einem zugehörigen Auswertgerät bestehen. Mit Hilfe von derartigen modernen Blutentnahmensystemen soll bei dem Patienten sowohl eine möglichst schmerzarme als auch eine einfach zu handhabende Blutentnahme gewährleistet werden. Im neueren Stand der Technik sind daher zahlreiche verschiedene Blutentnahmesysteme bekannt, die geeignet sind, die zur Blutgewinnung erforderlichen Stichwunden auf einfache Weise verhältnismäßig schmerzarm zu erzeugen. Derartige Blutentnahmensysteme beinhalten in der Regel ein Gehäuse mit einer Austrittsöffnung für die Spitze einer Lanzette sowie einen dem Gehäuse entlang einem vorbestimmten geraden Einstichweg beweglichen Lanzettenhalter zur Halterung der Lanzette. Der Lanzettenhalter wird bei der Einstich- und Rückführbewegung von einem Lanzettenantrieb bewegt, der ein elastisches Antriebselement, üblicherweise eine Metallfeder aufweist. Der Lanzettenhalter ist in einer ersten Position, in der sich das elastische Antriebselement in einem gespannten Zustand befindet, üblicherweise mittels einer Arretiervorrichtung arretiert. Nach dem Lösen einer derartigen Arretiervorrichtung entspannt sich das elastische Antriebselement, sodass die Bewegung des Antriebselementes in eine Einstichbewegung des Lanzettenantriebes umgesetzt wird, wobei die von dem Lanzettenhalter gehaltene Lanzette mit hoher Geschwindigkeit entlang dem vorbestimmten Einstichweg in eine Einstichrichtung bewegt wird, bis die Spitze der Lanzette aus der Austrittsöffnung des Blutentnahmesystems austritt. In einem gegen die Austrittsöffnung gedrückten Körperteil wird eine Wunde erzeugt. Unmittelbar danach erfolgt üblicherweise eine Rückführung der Lanzette durch den Lanzettenantrieb in das Gehäuse.

Sowohl die Antriebseinheiten der modernen Stechhilfen, die aufgrund einer hohen Einstichgeschwindigkeit eine Schmerzminimierung gewährleisten, als auch die Rückrührung der Lanzette nach dem Einstich tragen folglich bei heutigen Stechhilfen dafür Sorge, dass die Blutentnahme für den Patienten erheblich angenehmer verläuft.

Im Stand der Technik werden derartige Blutentnahmensysteme beispielhaft in den Dokumenten US 4,442,836, US 4,535,769 sowie US 4,924,879 beschrieben.
Bei der in dem Patent US 4,924,879 beschriebenen Konstruktion wirkt eine Spiralantriebsfeder auf ein Rad, dessen Drehung mittels eines mit dem Rad verbundenen Hebels in eine Einstich- und Rückführbewegung der Lanzette umgesetzt wird. Der Schmerz soll unter anderem dadurch vermindert werden, dass diese Bewegung sehr schnell abläuft. Die Konstruktion unter Verwendung präzis gefertigter Metallteile ist jedoch aufwendig und relativ voluminös. Außerdem ist nachteilig, dass die Lanzette beim Spannen des Lanzettenantriebs aus der Austrittsöffnung austritt und daraus eine Verletzungsgefahr für den Benutzer resultiert.

Das Patent EP 0 582 226 beschreibt ein weiteres Stechgerät, das zur Entnahme von Blutproben geeignet ist. Innerhalb des Stechgerätes ist ein Kolben beweglich gelagert, der durch Federmittel angetrieben wird. An einem Ende des Kolbens befindet sich eine Lanzette, die bei einem Stichvorgang aus einer hierfür vorgesehenen Öffnung des Gehäuses hinaustritt. Der Kolben besitzt weiterhin an seinem Außenumfang nur begrenzt haltbare Flügel, die auf der Gehäusewand des Stechgerätes aufliegen. Beim Ausführen des Stechvorgangs wird mittels der Federelemente der Kolben in Einstichrichtung bewegt, wobei die Flügel des Kolbens zerstört werden, sodass diese nicht länger auf dem Gehäuse aufliegen.

Nachteil des Standes der Technik ist, dass die Federelemente zum Antreiben des Kolbens hinreichende Kraft aufwenden müssen, um die Flügel des Kolbens zunächst zu zerstören, bevor der Kolben in Einstichrichtung bewegt werden kann. Des weiteren verursacht eine nicht vollständige Zerstörung der Flügel Reibungseffekte, wenn der Kolben innerhalb des Gehäuses in Einstichrichtung bewegt wird. Die Bedingungen eines Stechvorgangs werden somit in Abhängigkeit von den Reibungseffekten geändert, sodass keine definierten Kraftverläufe beim Stechvorgang wirken. Dies beeinflusst unter anderem die Einstichgeschwindigkeit der Lanzette in ein Hautteil, sodass der Patient mit unterschiedlichen Einstichschmerzen rechnen muss.

Des weiteren weist die Lanzette einen Sterilschutz auf, der vor Gebrauch von der Nadelspitze abgezogen werden muss. Aufgrund einer zu stark ausgeübten Zugbewegung besteht hierbei jedoch die Möglichkeit, die Flügel zu beschädigen, sodass ggf. ein Stechvorgang unbeabsichtigt ausgelöst wird. Da der beschriebene Mechanismus ausschließlich für Einmalstechhilfen geeignet ist und ein Wiederspannen der Lanzette aufgrund der zerstörten Flügel des Kolbens nicht möglich ist, muss die Lanzette folglich ungenutzt verworfen werden.

Weiterhin offenbart das Patent US 4,416,279 ein Blutentnahmesystem, bei dem durch eine Drehung eines äußeren Gehäuseteils der Lanzettenhalter entlang einer im Gehäuse befindlichen schräg angeordneten Rampe entgegen der Einstichrichtung bewegt wird. Aufgrund dieser Bewegung erfolgt eine Spannung eines Federteils, welches als Antriebselement für den Lanzettenhalter dient. Die in das Gehäuse hineinragende Schräge weist ein Plateau auf, auf dem der Lanzettenhalter nach einer ersten Drehbewegung des äußeren Gehäuseteils zwischengelagert werden kann. In dieser Position ist der Lanzettenhalter soweit entgegen der Einstichrichtung zurückgezogen, dass die Lanzette von dem Lanzettenhalter entnommen und ausgetauscht werden kann. Durch Fortführen der Drehbewegung wird der Lanzettenhalter weiter entlang der Schräge entgegen der Einstichrichtung bewegt, wobei das Federelement weiterhin gespannt wird, bis der Lanzettenhalter über die Schräge hinaus bewegt wird und durch die Federkraft in Einstichrichtung angetrieben wird. Die Lanzettenspitze tritt aus dem Gehäuse.

Eine Stechhilfe entsprechend dem Oberbegriff des Anspruchs 1 ist offenbart in Dokument WO 93/09723.

Nachteil des Standes der Technik ist es, dass das Handling des Blutentnahmesystems für den Benutzer durch die Drehbewegung des äußeren Gehäuseteiles erschwert wird. Um ein Spannen des Blutentnahmesystems sowie gleichzeitig eine Positionierung der Fingerkuppe an der Austrittsöffnung des Blutentnahmesystems zu ermöglichen, muss der Patient den Finger in einer dafür vorgesehenen Halterung positionieren. Die Halterung dient gleichzeitig dazu, das Blutentnahmesystem ortsfest auf der Fingerkuppe zu positionieren, während der Benutzer mit der anderen Hand das äußere Gehäuseteil dreht. Der Gebrauch des dargestellten Blutentnahmesystems erweist sich als äußerst unhandlich und ist vor allem für ältere Menschen kaum zu praktizieren.

Aufgabe der vorliegenden Erfindung ist es, ein Blutentnahmesystem sowie ein Verfahren zur Blutentnahme bereitzustellen, in dem die beschriebenen Nachteile des Standes der Technik vermieden werden. Das System sowie das Verfahren zeichnen sich insbesondere dadurch aus, dass eine einfache Handhabung gewährleistet wird. Durch eine schnelle Antriebsbewegung des Lanzettenhalters findet eine Minimierung des Einstichschmerzes statt. Der Aufbau des Blutentnahmesystems soll dabei insbesondere robust und kostengünstig sein.

Die Erfindung beinhaltet ein Blutentnahmesystem mit einem Gehäuse, in dem sich eine Öffnung befindet sowie einem Lanzettenhalter mit einer Lanzette, der in dem Gehäuse beweglich gelagert ist. Der Lanzettenhalter weist dabei mindestens ein Lagerelement auf. In dem Gehäuse ist weiterhin eine Auflagefläche angeordnet, die so positioniert ist, dass in einer ersten Position des Lanzettenhalters das Lagerelement auf der Auflagefläche aufliegt. Das Blutentnahmesystem beinhaltet weiterhin eine Auslöseeinheit mit einer Auslösetaste. Durch eine lineare Bewegung der Auslösetaste erfolgt mittels der Auslöseeinheit eine Überführung der linearen Bewegung in eine relative Drehbewegung des Lagerelementes und der Auflagefläche zueinander, wodurch der Lanzettenhalter in eine zweite Position überführt wird und von der Auflagefläche fällt. Ein Federelement des Blutentnahmesystems ist mit dem Lanzettenhalter verbunden und wird durch die Fallbewegung des Lagerelementes von einem zuvor gespannten Zustand in einen zumindest teilweise entspannten Zustand überführt. Durch die Bewegung des Lanzettenhalters relativ zum Gehäuse tritt die Spitze der Lanzette aus der Öffnung des Gehäuses hinaus, sodass in ein dort positioniertes Körperteil ein Stich ausgeführt werden kann.

Das erfindungsgemäße Blutentnahmesystem zeichnet sich durch einen einfachen, robusten Aufbau aus. Sowohl an das Lagerelement als auch an die Auflagefläche werden keine besonderen Anforderungen in Form oder Material gestellt. Im Sinne der Erfindung sind dabei vielfältige Gestaltungsmöglichkeiten denkbar, die eine Lagerung des Lanzettenhalters auf der Auflagefläche ermöglichen. Eine Herstellung der Stechhilfe mit hohen Toleranzen wird somit ermöglicht, ohne dass die Funktionsweise des Blutentnahmegerätes beeinflusst wird. Dies erlaubt eine Vereinfachung des Herstellungsprozesses, sodass die Produktionskosten der Stechhilfe gesenkt werden können. Besonders bei Einmalstechhilfen, die mit einer hohen Stückzahl hergestellt werden, ist dies ein entscheidender Vorteil.

Durch den dargestellten Mechanismus wird die Stichbewegung des Lanzettenhalters bzw. der Lanzette so schnell ausgeführt, dass eine Minimierung des Einstichschmerzes erfolgt. Sobald der Lanzettenhalter in Einstichrichtung von der Auflagefläche fällt, wird der Stechvorgang ausgelöst. Hierbei ist die im System gespeicherte potentielle Energie maximal. Anders als im Stand der Technik, bei dem z. B. der Stechvorgang durch Abbrechen von Häkchen ausgelöst wird, ist der wirkende Kraftmoment des Systems bei jedem Stechvorgang konstant, da z. B. zusätzliche Reibungseffekte, die durch ungleichmäßiges Abreißen der Häkchen verursacht werden, nicht auftreten. Dies gewährleistet einen Stechvorgang unter konstanten, reproduzierbaren Bedingungen.

Aufgrund der Überführung einer linearen Bewegung der Auslösetaste in eine Drehbewegung ist die Bedienung des Blutentnahmesystems besonders benutzerfreundlich, da der Patient beispielsweise durch ein Drücken der Auslösetaste die Stechhilfe betätigen kann und somit komplizierte Handhabungsschritte erspart bleiben.

Zum Auslösen eines Stechvorgangs sind prinzipiell vielfältige Ausführungsformen der Auslöseeinheit denkbar, die eine lineare Bewegung der Auslösetaste in eine relative Drehbewegung des Lagerelementes und der Auflagefläche überführen. Hierbei ist eine Überführung einer linearen Bewegung der Auslöstaste in eine Drehbewegung des Lanzettenhalters möglich.

In einer bevorzugten Ausführungsform erfolgt die lineare Bewegung der Auslösetaste senkrecht zur Ebene der Drehbewegung, sodass beispielsweise eine Bedienung des Blutentnahmesystems durch das Drücken der Auslösetaste entlang der Einstichrichtung erfolgt.

In einer vorteilhaften Ausführungsform erweist es sich weiterhin als sinnvoll, dass der Lanzettenhalter zwei Lagerelemente, die jeweils auf einer Auflagefläche des Gehäuses aufliegen, beinhaltet. Hierdurch wird eine symmetrische Lagerung des Lanzettenhalters durch eine einfache Gehäusekonstruktion gewährleistet. Es ist natürlich auch eine Ausführungsform denkbar, in der mehr als zwei Lagerelemente und mehrere Auflageflächen enthalten sind. Prinzipiell ist die Anzahl der Lagerelemente und der entsprechenden Auflageflächen nicht begrenzt, sodass üblicherweise eine Konstruktion gewählt wird, bei der eine optimierte Lagerung des Lanzettenhalters erfolgt bei günstigen Herstellungskosten.

Zur Überführung der linearen Bewegung in eine relative Drehbewegung des Lagerelementes und der Auflagefläche zueinander beinhaltet die Auslöseeinheit ein Drehantriebselement, dass durch die lineare Bewegung der Auslösetaste gegen das Lagerelement gedrückt wird, sodass das Lagerelement von der Auflagefläche fällt. Beispielsweise weist das Drehantriebselement hierfür einen elastischen Stößel auf, so wie die Auflagefläche eine Vertiefung beinhalten kann, in der das Lagerelement des Lanzettenhalters gelagert wird. Durch eine lineare Bewegung der Auslösetaste wird der elastische Stößel von oben gegen das Lagerelement des Lanzettenhalters gedrückt und hierbei deformiert. Befindet sich das Lagerelement in einer Vertiefung der Auflagefläche, kann der Stößel aufgrund der Deformation auch unterhalb des Lagerelementes gelangen und dieses aus der Vertiefung der Auflagefläche raushebeln. Der Lanzettenhalter fällt hierdurch von der Auflagefläche und ein Stechvorgang wird ausgelöst.

Aufgrund der Fallbewegung des Lagerelementes und somit des Lanzettenhalters wird das Federelement entspannt. Hierbei ist im Sinne der Erfindung auch ein Prozess zu verstehen, in dem das Federelement einen entspannten Zustand nur durchläuft und anschließend wieder in einen gespannten Zustand überführt wird.

Beispielsweise kann die Fallbewegung des Lanzettenhalters durch einen hierfür vorgesehenen Anschlag gestoppt werden, sodass ausschließlich ein definierter Teil der Lanzette aus dem Gehäuse hervortritt. Der Anschlag, der folglich die Einstichtiefe des Blutentnahmesystems bestimmt, kann hierbei vorteilhafterweise Bestandteil einer hierfür vorgesehenen Kappe sein, die beweglich an das Gehäuse des Systems montiert ist, oder ist Bestandteil des Gehäuses selbst. Hierbei ist es denkbar, dass die Kappe in oder entgegen die Einstichrichtung beweglich am Gehäuse befestigt ist, sodass bei einem Anschlag am Gehäuse der Abstand zwischen Anschlag und Austrittsöffnung der Kappe variiert wird. Ist der Anschlag Bestandteil der Kappe, kann sowohl durch eine Verschiebung der Kappe in oder entgegen der Einstichrichtung die Position des Anschlages selbst und somit die Einstichtiefe verändert werden, als auch durch eine Drehbewegung der Kappe, bei der die axiale Position der Kappe konstant bleibt. Bei einer derartigen Ausführungsform beinhaltet die Kappe mehrere Anschläge mit unterschiedlichen Höhen, die in Abhängigkeit von der Drehbewegung der Kappe zum Einsatz kommen und die Einstichtiefe definieren. Ein derartiges System zur Regulierung der Einstichtiefe wird beispielsweise in dem Dokument EP 1 142 534 beschrieben.

Nachdem der Lanzettenhalter auf den Anschlag aufgeschlagen ist, wird dieser üblicherweise, wie beschrieben, mittels eines Rückholmechanismus in entgegengesetzte Richtung zurückbefördert, sodass die Lanzettenspitze nicht länger aus der Gehäuseöffnung hinausragt.

Prinzipiell ist das dargestellte System sowohl für Einmalstechhilfen als auch für Stechhilfen zur mehrfachen Verwendung geeignet. Das System befindet sich bevorzugt in einem gespannten Zustand, sobald das Lagerelement auf der Auflagefläche aufliegt. Dieser Zustand erweist sich insbesondere bei Einmalstechhilfen als besonders günstig, da eine Vorrichtung zum Spannen des Federelementes hierbei oftmals nicht vorgesehen ist. Einmalstechhilfen werden dann bereits in einem gespannten Zustand an den Kunden geliefert. Der Kunde muss hierbei ausschließlich nur noch die Auslösetaste betätigen, wodurch der Stechvorgang initiiert wird.

Um Kontaminationen zu vermeiden, ist eine Wiederverwendung der Einmalstechhilfe nicht erwünscht und wird sogar oftmals aktiv unterbunden. Dies kann zum einen durch das Fehlen einer Spannvorrichtung in der Einmalstechhilfe verwirklicht werden, wobei sich hierdurch der Aufbau der Stechhilfe vereinfacht. Zum anderen ist jedoch auch ein Mechanismus denkbar, der aktiv die Lanzette nach ihrer Verwendung blockiert. Hierbei kann, um ein missbräuchliches Wiederspannen zu vermeiden, beispielsweise das Lagerelement vom Federelement nach dem Stechvorgang von unten gegen die Auflagefläche gedrückt werden. Eine Positionierung des Lagerelementes auf der Auflagefläche wird bei dieser Ausführungsform somit verhindert. Generell sind vielfältige Ausführungsform denkbar, die ein Wiederspannen einer Einmalstechhilfe verhindert.

Soll das Blutentnahmesystem zur mehrfachen Verwendung geeignet sein, beinhaltet das System eine Vorrichtung zum Spannen des Antriebselementes, sodass die Stechhilfe nicht im gespannten Zustand ausgeliefert werden muss. Um einen unnötige Materialbelastung des Lagerelementes und der Auflagefläche zu vermeiden, liegt das Antriebselement in einer weiteren vorteilhaften Ausführungsform bei Auflage des Lagerelementes auf der Auflagefläche im entspannten oder zumindest teilweise entspannten Zustand vor. Vorteilhafter Weise erfolgt dann vor oder mit Betätigung der Auslöseeinheit zunächst ein Spannen des Antriebselementes bevor eine relative Drehbewegung des Lanzettenhalters und der Auflagefläche initiiert wird. Um Materialermüdungen bei Einmalstechhilfen ebenfalls zu vermeiden, ist ein derartiger Mechanismus natürlich auch hier anwendbar. Es kann sich jedoch auch als vorteilhaft erweisen, die Materialeigenschaften der Lagerelemente besonders robust auszuwählen, sodass die Lagerelemente der Belastung in einem gespannten Zustand problemlos widerstehen können. Die auf die Lagerelemente wirkende Spannung kann dann entsprechend groß gewählt werden, ohne dass es zu einer Beschädigung der Lagerelemente kommt. Entsprechend der vorliegenden Spannung ist es möglich, die Einstichgeschwindigkeit der Stechhilfe zu erhöhen, sodass eine Minimierung des Einstichschmerzes resultiert.

Ein weiterer wichtiger Aspekt bei der Konstruktion einer Stechhilfe ist, ein unbeabsichtigtes Auslösen eines Stechvorgangs, wie es insbesondere beim Abnehmen eines Sterilschutzes zu berücksichtigen ist, zu vermeiden. Hierbei sind verschiedene Mechanismen denkbar, die einen Verbleib der Lanzette im Gehäuse gewährleisten. Der Benutzer wird somit vor Verletzungen durch versehentliches Auslösen der Stechhilfe geschützt. Des weiteren wird durch derartige Verriegelungsmechanismen die Sterilität der Lanzette vor dem Stechvorgang gewährt. Bevorzugte Ausführungsformen eines Verriegelungsmechanismus können hierbei sowohl Verriegelungselemente als Bestandteil der Auslöseeinheit beinhalten, durch die eine relative Drehbewegung des Lagerelementes und der Auflagefläche zueinander verhindert wird, solange die Auslösetaste nicht betätigt wird. Derartige Verriegelungselemente können jedoch auch Bestandteile des Stechhilfengehäuses sein oder sich an einem abnehmbaren Sterilschutz, der zur sterilen Aufbewahrung der Lanzette geeignet ist, befinden. Es ist natürlich auch eine Kombination der beschriebenen Merkmale denkbar, sodass durch ein Zusammenwirken von einem oder mehreren Verriegelungselementen z. B. von Lanzettenhalter und Gehäuse ein unbeabsichtigtes Auslösen des Systems vorgebeugt wird. Hierbei kann sich einer der beschriebenen Ausführungsformen in Abhängigkeit von weiteren Funktionen der Stechhilfe, wie z. B. einer Einstichtiefenregulierung von Vorteil erweisen. Ist der Verriegelungsmechanismus Bestandteil des Sterilschutzes, wird eine relative Drehbewegung des Lagerelementes und der Auflagefläche so lange verhindert, wie der Sterilschutz nicht von der Lanzette gelöst ist. Beispielsweise beinhaltet der Sterilschutz hierbei Widerhaken, die eine Bewegung des Lanzettenhalters parallel zur Einstichrichtung verhindert. Weist die Auflagefläche vorteilhafterweise eine Vertiefung auf, kann ein Rausheben der Lagerelemente aus der Vertiefung der Auflagefläche verhindert werden. Eine derartige Ausführungsform erweist sich z. B. bei einer Einstichtiefenregulierung, bei der die axiale Position der Kappe festgelegt und nicht veränderbar ist, als besonders vorteilhaft.

Weiterhin ist offenbart ein Verfahren zum Auslösen eines Stechvorgangs mit einer Stechhilfe. Hierbei liegt zunächst ein Lanzettenhalter auf einer Auflagefläche im Inneren eines Gehäuses einer Stechhilfe auf. Durch den Benutzer wird eine Auslöseeinheit der Stechhilfe mittels einer linearen Bewegung betätigt. Hierbei wird die lineare Bewegung zumindest eines Teiles der Auslöseeinheit in eine relative Drehbewegung des Lanzettenhalters und der Auflagefläche zueinander überführt, bis der Lanzettenhalter von der Auflagefläche fällt und eine Fallbewegung ausführt. Aufgrund der Fallbewegung tritt eine Spitze einer Lanzette des Lanzettenhalters durch eine Öffnung des Gehäuses, sodass ein Stechvorgang ausgeführt werden kann.

Bevorzugte Ausführungsform des Verfahrens ergeben sich wie bereits beschrieben.

In Nachfolgendem wird anhand der Figuren beispielhaft die Erfindung verdeutlicht.
- Figur 1:: Seitenansicht eines Blutentnahmesystems
- Figur 2:: Aufsicht auf dem Lanzettenhalter innerhalb des Gehäuses
- Figur 3:: Auslösen eines Stechvorganges
- Figur 4:: Mechanismus zur Regulierung der Einstichtiefe
- Figur 5:: Verriegelungsmechanismus zur Vermeidung eines unbeabsichtigten Stechvorgangs

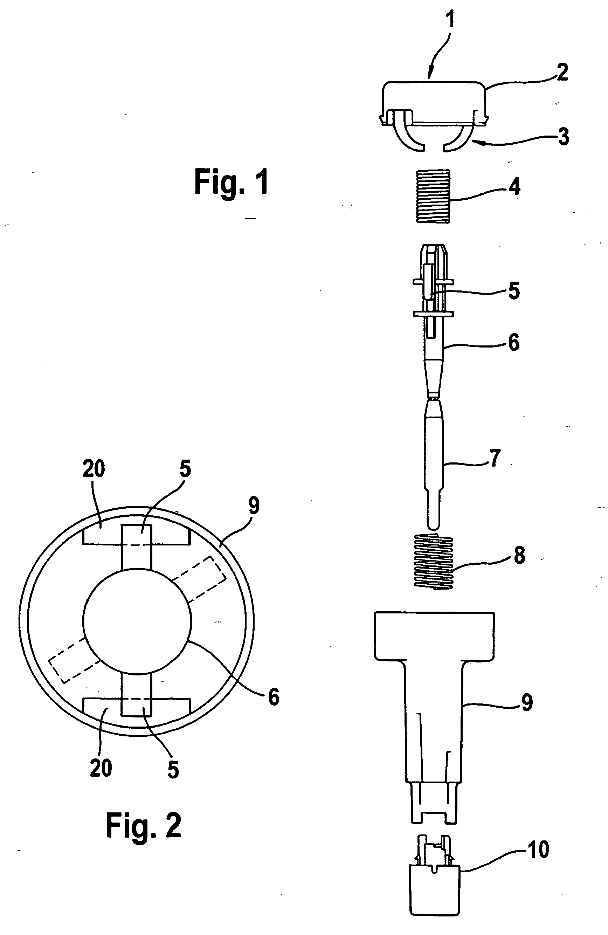

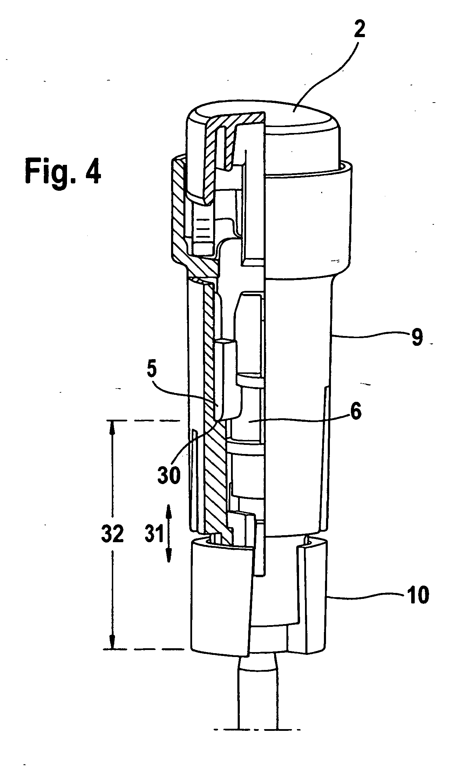

Figur 1 verdeutlicht anschaulich Bestandteile des Blutentnahmesystems vor deren Zusammensetzung. Das Blutentnahmesystem verfügt über ein Gehäuse 9, in dem der Lanzettenhalter hineingeführt wird. Der Lanzettenhalter wird im Gehäuse beweglich gelagert, sodass er bei einem Stechvorgang entlang der Einstichrichtung geführt werden kann. Hierfür beinhaltet das System weiterhin Antriebselemente, die mit dem Lanzettenhalter verbunden sind und auf diese wirken. Die Antriebselemente werden im gezeigten Beispiel durch jeweils eine Feder verwirklicht (4 und 8). Hierbei kann im Sinne der Erfindung, wie in Figur 1 dargestellt, der Lanzettenhalter lediglich lose auf einem Federelement aufliegen oder an diesem anliegen., ohne dass eine feste Verbindung zwischen Federelement und Lanzettenhalter besteht. Im Sinne der Erfindung ist die Verbindung des Lanzettenhalters mit einem Antriebselement dadurch charakterisiert, dass eine Kraft vom Antriebselement auf den Lanzettenhalter übertragen werden kann. Es sind aber auch Ausführungsformen möglich, bei denen der Lanzettenhalter fest mit einem Antriebselement verbunden ist. Der Lanzettenhalter ist darüber hinaus derartig im Gehäuse positioniert, dass er während des Stechvorgangs senkrecht zur Einstichrichtung eine Rotation ausführen kann, wie es anhand nachfolgender Figuren noch veranschaulicht wird. Der Lanzettenhalter selbst weist einen Sterilschutz (7) auf, der über eine Lanzette gestülpt ist, um eine Sterilität der Lanzette vor deren Gebrauch zu gewährleisten sowie eventuelle unbeabsichtigte Verletzungen durch die Lanzettenspitze zu vermeiden. Zunächst wird die Feder (8) in das Gehäuse (9) eingeführt. Bei einem späteren Gebrauch der Stechhilfe wird somit ein Rücktransport der Lanzette in das Gehäuse nach dem Stechvorgang bewirkt. Die Feder (8) wird auch als Rückholfeder bezeichnet. Der Lanzettenhalter wird innerhalb der Feder (8) sowie dem Gehäuse (9) eingeführt. Der Lanzettenhalter verfügt weiterhin über Lagerelemente (5), die innerhalb des Gehäuses auf dafür vorgesehene Auflageflächen (nicht dargestellt) aufliegen. Sowohl die Feder (4) als auch die Feder (8) wirken in der Weise mit dem Lanzettenhalter zusammen, dass beim Aufliegen der Lagerelemente (5) auf den Auflageflächen innerhalb des Gehäuses (9) die Federn (4) (Antriebsfeder) gespannt ist, wobei die Feder (8) (Rückholfeder) entspannt vorliegt. Während des Stechvorgangs entspannt sich die Antriebsfeder (4) unter Beschleunigung des Lanzettenhalters. Dieser führt eine Fallbewegung aus, wobei er auf die Rückholfeder (8), die dadurch gespannt wird, trifft. Der Lanzettenhalter fällt innerhalb des Gehäuses bis zu einem Anschlag. Von der dann voll gespannten Rückholfeder (8) wird der Lanzettenhalter wieder zurückgezogen. Zu diesem Zeitpunkt liegt die Antriebsfeder (4) entspannt vor. Nach dem Stich und vor einem eventuellen Wiederspannen der Stechhilfe sind beide Federn (4 und 8) vollständig entspannt und liegen an dem Lanzettenhalter an. Im unteren Ende des Gehäuses (9) wird eine Kappe (10) an dem Gehäuse beweglich montiert, sodass mittels einer Drehbewegung der Kappe der Betrag der Lanzettenspitze, der aus dem Gehäuse austritt, verändert werden kann. Aufgrund dieses veränderbaren Abstandes können unterschiedliche Einstichtiefen der Lanzette eingestellt werden. Die Regelung der Einstichtiefe wird im Folgenden beispielhaft anhand von Figur 4 noch einmal dargestellt. Im oberen Ende des Gehäuses verschließt die Auslöseeinheit (1) die obere Gehäuseöffnung. Im dargestellten Beispiel beinhaltet die Auslöseeinheit eine Auslösetaste (2) sowie zwei elastische Häkchen (3) als Drehantriebselemente. Wird die Auslösetaste (2) entlang der Einstichrichtung der Lanzette betätigt, verursachen die Häkchen (3) eine Drehbewegung des Lanzettenhalters.

Figur 2 zeigt eine Aufsicht der Stechhilfe, bei der sich der Lanzettenhalter (6) in dem Lanzettengehäuse (9) in einer ersten Position befindet. Der Lanzettenhalter (6) verfügt in dem dargestellten Beispiel über zwei Lagerelemente (5), die in Form von Haltearmen ausgebildet sind. Die Haltearme (5) liegen in der ersten Position auf den Auflageflächen (20) des Gehäuses (9) auf. Durch eine Rotationsbewegung des Lanzettenhalters (6) werden die Lagerelemente in einer zweiten Position (gestrichelt dargestellt) bewegt, in der sie nicht mehr auf den Auflageflächen aufliegen. Der mit den Lagerelementen verbundene Lanzettenhalter kann nun eine Bewegung in Einstichrichtung ausführen.
Durch die Bewegung des Lanzettenhalters in Einstichrichtung wird ein Stechvorgang ausgeführt.

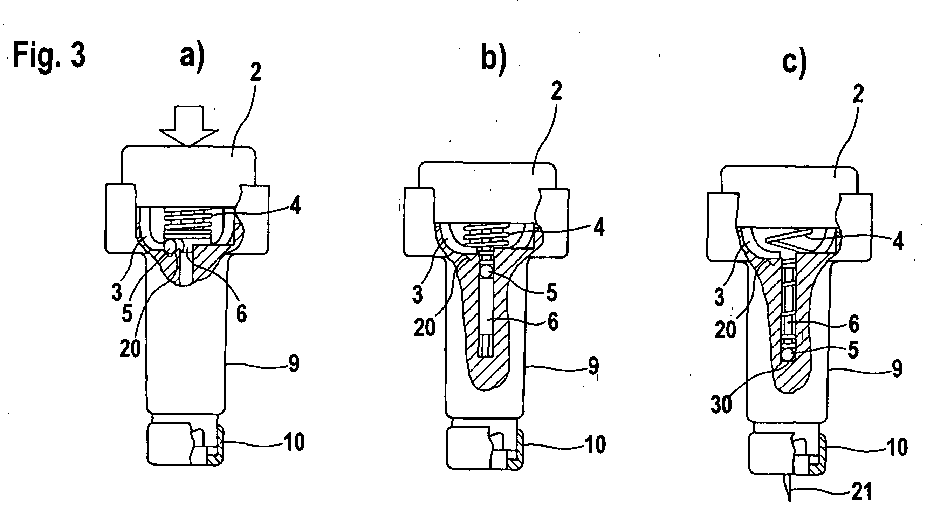

Figur 3 verdeutlicht das Auslösen eines Stechvorgangs anhand einer Stechhilfe, die - wie bereits dargestellt - als Drehantriebselemente einen elastischen Haken aufweist.

Figur 3a zeigt das Gehäuse (9) der Stechhilfe, wobei durch eine schematisch dargestellte unterbrochene Gehäusewand in das Gehäuseinnere Einblick genommen werden kann. Das Gehäuse (9) wird von einer Auslösetaste (2) sowie einer Kappe (10) verschlossen. Mit der Auslösetaste (2) ist ein Haken (3) als Drehantriebselement verbunden. Im Gehäuseinneren befindet sich der Lanzettenhalter (6), der durch eine Feder (4) angetrieben wird. Der Lanzettenhalter (6) verfügt über ein Lagerelement in Form eines geknickten Stabes (5), mittels der der Lanzettenhalter (6) auf einer Auflagefläche (20) des Gehäuses (9) gelagert wird. Die Auflagefläche (20) des Gehäuses weist eine Mulde auf, die eine Positionierung des Lagerelementes auf der Auflagefläche erleichtert. Beim Aufliegen der Lagerelemente (5) auf der Auflagefläche (20) befindet sich die Feder (4) im komprimierten Zustand. Das Drehantriebselement (3) ist zu diesem Zeitpunkt neben dem Lagerelement positioniert. Durch ein Drücken der Auslösetaste (2) entlang zur Einstichrichtung - wie durch den Pfeil in Figur 3a angedeutet wird - erfolgt das Auslösen des Stechvorgangs. Hierbei wird das elastische Häkchen (3) beim Hinunterdrücken der Auslösetaste deformiert und greift in die Mulde der Auflagefläche (20) ein, sodass der Lanzettenhalter eine Rotation ausführt und das Lagerelement (5), wie in Figur 3b dargestellt, von der Auflagefläche fällt. Aufgrund der Fallbewegung bewegt sich der Lanzettenhalter (6) in Einstichrichtung, wobei die Feder (4) entspannt wird. Die Fallbewegung des Lanzettenhalters erfolgt bis zu einem Anschlag (30) innerhalb des Gehäuses (9), auf dem das Lagerelement (5) aufschlägt, sodass die Fallbewegung des Lanzettenhalters gestoppt wird. Hierbei tritt die Lanzettenspitze (21) aus einer Öffnung (nicht dargestellt) des Gehäuses aus. Der Betrag der Lanzettenspitze (21), der aus dem Gehäuse austritt, ist über die Kappe (10) variierbar, sodass die Einstichtiefe des Blutentnahmesystems - wie beschrieben - eingestellt werden kann. Mittels der Rückholfeder (8) wird die Lanzettenspitze wieder in das Gehäuse zurückgeholt, sodass die Lanzette nach einer Perforation einer Hautstelle nicht in dem Körperteil verbleibt.

Figur 4 zeigt ein Blutentnahmesystem mit einem analogen Aufbau, wie er bereits in Figur 1 bis 3 dargestellt ist. Anhand des einseitig offen dargestellten Systems wird beispielhaft ein Mechanismus zur Regulierung der Einstichtiefe dargestellt. Die in Figur 4 gezeigte Stechhilfe veranschaulicht einen Zustand, in dem die Lanzette auf dem unteren Ende des Gehäuses (9) hervorragt. Nachdem der Stechvorgang durch die Auslösetaste (2) initiiert wurde, fällt der Lanzettenhalter (6) entlang der Einstichrichtung. Hierbei findet eine Fallbewegung statt, bis der Lanzettenhalter (6) an einem Anschlag (30) des Gehäuses (9) anstößt, sodass die Fallbewegung gestoppt wird. Im gezeigten Beispiel wird das Lagerelement (5), das zur Lagerung des Lanzettenhalters auf den Auflageflächen (20) verwendet wird, ebenfalls als Widerlager hinsichtlich des Anschlages (20) verwendet. Es sind folglich keine zusätzlichen baulichen Maßnahmen notwendig, um die Fallbewegung des Lanzettenhalters zu definieren. Bei der dargestellten Stechhilfe ist der Anschlag (30) Bestandteil des Gehäuses (9) und nicht veränderbar. Unter diesen Bedingungen ist die Fallstrecke, entlang sich der Lanzettenhalter bewegt, unabhängig von der gewählten Einstichtiefe. Dies gewährleistet ein konstantes Kraftmoment bei jedem Stechvorgang. Zur Veränderung der Einstichtiefe wird die Kappe (10), die beweglich am Gehäuse (9) montiert ist, entlang oder gegen die Einstichrichtung bewegt, wie es durch den Pfeil (31) in der Figur 4 veranschaulicht wird. Der Abstand zwischen dem Anschlag (30) des Gehäuses (9) und der Öffnung der Kappe (10), aus der die Lanzette austritt, ist somit durch eine Drehbewegung der Kappe (10) veränderbar. Der Abstand zwischen Anschlag (30) und der Öffnung wird in Figur 4 durch den Pfeil (32) gekennzeichnet. In Abhängigkeit dieses Abstandes wird folglich der Betrag der Lanzette, der aus dem Gehäuse rausragt, definiert. Bei der Veränderung der Einstechtiefe sind mehrere Möglichkeiten denkbar, die die Kappe entlang der Strecke (31) positionieren. Beispielsweise wird dieses durch eine Drehbewegung der Kappe relativ zum Gehäuse verwirklicht. Es ist jedoch auch denkbar, dass die Kappe (10) direkt durch ein Ziehen oder Drücken in Richtung (31) auf den Anschlag (30) hin oder von diesem weg bewegt wird. Allgemein kann die Positionierung der Kappe (10) stufenlos oder in definierten Abständen erfolgen. Weiterhin erweist es sich als vorteilhaft, dass die Stechhilfe Verrasterungselemente (nicht dargestellt) aufweist, die die Kappe in einer vorbestimmten Position halten, sodass diese nicht durch das Auflegen der Kappe (10) auf ein Körperteil verschoben wird. In einer vorteilhaften Ausführungsform wird dem Benutzer mittels einer Skala die resultierende Einstichtiefe der Lanzette angezeigt. Prinzipiell sind vielfältige Möglichkeiten von Mechanismen zur Einstellung der Einstichtiefe denkbar.

Beispielsweise wird ein Mechanismus zur Regulierung der Einstichtiefe in dem Patent US 4,895,147 beschrieben. Hierbei wird die Einstichtiefe des Blutentnahmesystems durch die Verschiebung eines Anschlages des Lanzettengehäuses entlang oder gegen die Einstichrichtung der Stechhilfe eingestellt. Der Lanzettenhalter ist dabei mit einem Kontrollelement verbunden, der beim Auslösen eines Stechvorgangs gegen den Anschlag fällt, sodass die Fallbewegung des Lanzettenhalters gestoppt wird. Die Länge der Strecke, entlang der Lanzettenhalter fällt, wird bei einem derartigen Mechanismus in Abhängigkeit von der Position des verschiebbaren Anschlages bestimmt. In Abhängigkeit zur Einstichtiefe resultieren folglich unterschiedlich wirkende Kraftmomente.

Das Dokument US 6,056,765 offenbart einen weiteren Mechanismus zur Veränderung der Einstichtiefe eines Blutentnahmesystems. Hierbei ist die Lanzette in dem Lanzettenhalter verschiebbar gelagert. Eine Veränderung der Einstichtiefe wird erreicht, indem die Nadel unterschiedlich weit in den Lanzettenhalter eingeführt wird, wobei der Anschlag für den Lanzettenhalter ortsfest im Gehäuse positioniert ist.

Prinzipiell sind vielfältige Möglichkeiten zur Regulierung der Einstichtiefe denkbar, da das Blutentnahmesystem aufgrund seines Auslösemechanismus keine weiteren Bedingungen an die Stechhilfe stellt, die eine Beschränkung für derartige Mechanismen darstellen würden.

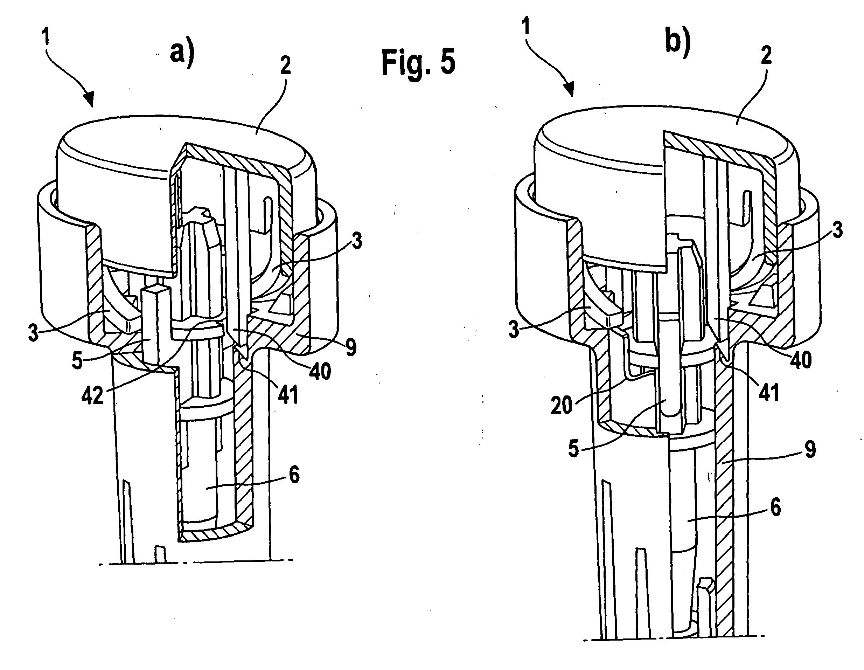

Figur 5 veranschaulicht beispielhaft einen Verriegelungsmechanismus, der ein unbeabsichtigtes Auslösen eines Stechvorgangs verhindert. Figur 5 stellt den oberen Bereich des Gehäuses (9) dar, in dem das Auslösen eines Stechvorgangs bewirkt wird. Um ein unbeabsichtigtes Auslösen zu verhindern, weist die Auslöseeinheit (1) neben der Auslösetaste (2) und der Drehantriebselemente (3) ein Verriegelungselement (40) auf. Das Verriegelungselement (40) ist in Form eines Stabs ausgebildet, der ins Gehäuseinnere reicht. Wird die Auslösetaste nicht betätigt, ist der Stab (40) zwischen dem Gehäuse (9) und dem Lanzettenhalter (6) positioniert. Der Lanzettenhalter (6) verfügt hierbei über eine Einkerbung (42), in der das Verriegelungselement (40) eingreift. Aufgrund der beschriebenen Positionierung wird eine Rotation des Lanzettenhalters verhindert. Erst beim Drücken der Auslösetaste in Einstichrichtung wird das Verriegelungselement (40) gegen eine Schräge (41) des Gehäuses (9) gedrückt. Durch weiteres Drücken der Auslösetaste wird das Verriegelungselement entlang der Schräge (41) geführt und dabei nach außen gespreizt. Die seitliche Dehnung des Verriegelungselements erfolgt dabei in dem Maße, dass der Stab nicht länger in die Kerbe (42) des Lanzettenhalters eingreift. Gleichzeitig werden die Halterarme (3) gegen die Lagerelemente (5) gedrückt und bewirken eine Rotation des Lanzettenhalters, der zu diesem Zeitpunkt nun nicht mehr vom Verriegelungselement (40) blockiert wird. Die Rotation erfolgt solange, bis das Lagerelement (5) von der Auflagefläche des Gehäuses hinunterfällt und ein Stechvorgang ausgelöst wird. Hierbei kann die Auflagefläche (20) - wie bereits gezeigt - eine Vertiefung aufweisen, in der Lagerelement positioniert wird. Es ist jedoch auch denkbar, dass auf eine derartige Vertiefung der Auflagefläche verzichtet wird, da aufgrund des Verriegelungselementes eine sichere Lagerung der Lagerelemente auf der Auflagefläche gewährleistet wird. Verzichtet man auf eine derartige Vertiefung der Auflagefläche, hat dies z. B. den Vorteil, dass bei der Herstellung des Blutentnahmesystems größere Toleranzen geduldet werden können, da eine exakte Abstimmung des Lanzettenhalters auf das Gehäuse in dem Maße nicht mehr erforderlich ist. Die Produktionskosten eines solchen Blutentnahmesystems werden somit minimiert.

Der dargestellte Verriegelungsmechanismus ist neben den bereits genannten Verriegelungssystemen als eine vorteilhafte Ausführungsform zur Veranschaulichung dargestellt. Prinzipiell sind jedoch jegliche Ausführungsformen denkbar, die ein unbeabsichtigtes Auslösen eines Stechvorgangs verhindern. Dies wird vorteilhafterweise dadurch bewerkstelligt, dass eine Rotation des Lanzettenhalters relativ zu den Auflageflächen blockiert wird.

## Patentansprüche

1. Stechhilfe beinhaltend
- ein Gehäuse (9) mit einer Öffnung, in dem ein Lanzettenhalter (6) mit einer Lanzette beweglich gelagert ist,
- wobei der Lanzettenhalter (6) mit einem Federelement verbunden ist und der Lanzettenhalter (6) mindestens ein Lagerelement (5) beinhaltet, und
- in dem Gehäuse (9) mindestens eine Auflagefläche (20) in der Weise angeordnet ist, dass das Lagerelement (5) in einer ersten Position des Lanzettenhalters (6) auf der Auflagefläche (20) aufliegt, sowie
- eine Auslöseeinheit (1), durch die der Lanzettenhalter (6) in eine zweite Position überführt wird, wobei eine Auslösetaste (2) der Auslöseeinheit (1) eine lineare Bewegung ausführt und die lineare Bewegung in eine relative Drehbewegung des Lagerelementes (5) und der Auflagefläche (20) zueinander überführt wird, so daß das Lagerelement (5) in der zweiten Position des Lanzettenhalters (6) von der Auflagefläche (20) falls, wobei sich das vor der Fallbewegung gespannte Federelement zumindest teilweise entspannt und den Lanzettenhalter (6) relativ zum Gehäuse (9) bewegt, so daß die Spitze (21) der Lanzette aus der Offnung des Gehäuses (9) austritt, **dadurch gekennzeichnet, dass** die Auslöseeinheit (1) ein Drehantriebselement (3) beinhaltet, das durch die lineare Bewegung der Auslösetaste (2) gegen das Lagerelement (5) gedrückt wird, sodass das Lagerelement (5) von der Auflagefläche (20) fällt.

2. Stechhilfe gemäß Anspruch 1,
bei der das Federelement in der Weise mit dem Lanzettenhalter (6) verbunden ist, dass sich das Federelement beim Aufliegen des Lagerelementes (5) auf der Auflagefläche (20) im gespannten Zustand befindet.

3. Stechhilfe gemäß Anspruch 1,
bei der die lineare Bewegung der Auslösetaste (2) entlang einer Längsachse des Gehäuses (9) erfolgt.

4. Stechhilfe gemäß Anspruch 1,
bei der das Drehantriebselement (3) mindestens einen elastischen Stößel beinhaltet, der durch die Bewegung der Auslösetaste (2) deformiert wird,

5. Stechhilfe gemäß Anspruch 1,
bei der das Drehantriebselement (3) mindestens eine schräge, in das Gehäuseinnere reichende Rampe aufweist.

6. Stechhilfe gemäß Anspruch 1,
bei der das Lagerelement (5) von dem Federelement oder einem weiteren Federelement nach dem Austritt der Lanzette aus der Öffnung des Gehäuses (9) gegen eine Unterseite der Auflagefläche (20) gedrückt wird.

7. Stechhilfe gemäß Anspruch 1,
bei der der Lanzettenhalter (6) zwei Lagerelemente (5) beinhaltet,

8. Stechhilfe gemäß Anspruch 1,
bei der die Auflagefläche (20) eine Vertiefung aufweist, in der das Lagerelement (5) des Lanzettenhalters (6) gelagert wird.

9. Stechhilfe gemäß Anspruch 1,
bei der die Auslöseeinheit (1) ein Verriegelungselement (40) aufweist, durch das eine relative Drehbewegung des Lagerelements (5) und der Auflagefläche (20) verhindert wird, solange die Auslösetaste (2) nicht betätigt wird.

10. Stechhilfe gemäß Anspruch 9,
bei der das Verriegelungselement (40) in eine Ausnehmung des Lanzettenhalters (6) eingreift.

11. Stechhilfe gemäß Anspruch 1 oder 9,
bei der das Gehäuse (9) ein Verriegelungselement (40) aufweist, durch das eine unbeabsichtigte, relative Drehbewegung des Lagerelements (5) und der Auflagefläche (20) verhindert wird.

12. Stechhilfe gemäß Anspruch 1,
bei der die Stechhilfe einen abnehmbaren Sterilschutz (7) für die Lanzette aufweist, der ein Verriegelungselement (40) beinhaltet, durch das eine relative Drehbewegung des Lagerelementes (5) und der Auflagefläche (20) verhindert wird, solange der Sterilschutz (7) nicht von der Lanzette gelöst ist.

13. Stechhilfe gemäß Anspruch 12,
bei der der Sterilschutz (7) einen Widerhaken beinhaltet, der eine Bewegung des Lanzettenhalters (6) parallel zur Einstichrichtung der Lanzette verhindert.

14. Stechhilfe gemäß Anspruch 1,
bei der das Gehäuse einen Anschlag (30) aufweist, durch den die Fallbewegung des Lanzettenhalters (6) gestoppt wird

15. Stechhilfe gemäß Anspruch 15,
bei der die Fallbewegung des Lanzettenhalters (6) durch ein Zusammenwirken des Anschlages (30) und mindestens eines Lagerelementes (5) gestoppt wird.

16. Stechhilfe gemäß Anspruch 1,
bei der sich die Öffnung des Gehäuses (9) in einer Kappe (10) befindet, die beweglich an dem Gehäuse (9) montiert ist.

17. Stechhilfe gemäß einem der Ansprüche 1 bis 17, **dadurch gekennzeichnet, dass** sie keine Vorrichtung zum Spannen des Federelementes aufweist.

18. Stechhilfe gemäß einem der Ansprüche 1 bis 18, **dadurch gekennzeichnet, dass** sie einen Mechanismus aufweist, der aktiv die Lanzette nach ihrer Verwendung blockiert.

## Claims

1. Lancing aid comprising
- a housing (9) with an opening in which a lancet holder (6) with a lancet is mounted in a movable manner,
- wherein the lancet holder (6) is connected to a spring element and the lancet holder (6) comprises at least one bearing element (5), and
- at least one supporting surface (20) is arranged in the housing (9) in such a manner that the bearing element (5) rests on the supporting surface (20) in a first position of the lancet holder (6), as well as
- a triggering unit (1) which transfers the lancet holder (6) into a second position, where a trigger button (2) of the triggering unit (1) makes a linear movement and the linear movement is converted into a relative rotational movement of the bearing element (5) and of the supporting surface (20) relative to each other so that the bearing element (5) falls from the supporting surface (20) in the second position of the lancet holder (6), during which the spring element that is tensioned before the falling movement at least partially relaxes and moves the lancet holder (6) relative to the housing (9) such that the tip (21) of the lancet emerges from the opening of the housing (9), **characterized in that** the triggering unit (1) comprises a rotary drive element (3) which is pressed against the bearing element (5) by the linear movement of the trigger button (2) so that the bearing element (5) falls from the supporting surface (20).

2. Lancing aid according to claim 1,
in which the spring element is connected to the lancet holder (6) in such a manner that the spring element is in a tensioned state when the bearing element (5) rests on the supporting surface (20).

3. Lancing aid according to claim 1,
in which the linear movement of the trigger button (2) takes place along a longitudinal axis of the housing (9).

4. Lancing aid according to claim 1,
in which the rotary drive element (3) comprises at least one elastic push rod which is deformed by the movement of the trigger button (2).

5. Lancing aid according to claim 1,
in which the rotary drive element (3) has at least one slanted ramp which extends into the interior of the housing.

6. Lancing aid according to claim 1,
in which the bearing element (5) is pressed against an underside of the supporting surface (20) by the spring element or an additional spring element after the lancet emerges from the opening of the housing (9).

7. Lancing aid according to claim 1,
in which the lancet holder (6) comprises two bearing elements (5).

8. Lancing aid according to claim 1,
in which the supporting surface (20) has a depression which bears the bearing element (5) of the lancet holder (6).

9. Lancing aid according to claim 1,
in which the triggering unit (1) has a latching element (40) which prevents a relative rotational movement of the bearing element (5) and the supporting surface (20) as long as the trigger button (2) is not actuated.

10. Lancing aid according to claim 9,
in which the latching element (40) engages in a recess of the lancet holder (6).

11. Lancing aid according to claim 1 or 9,
in which the housing (9) has a latching element (40) which prevents an inadvertent relative rotational movement of the bearing element (5) and the supporting surface (20).

12. Lancing aid according to claim 1,
in which the lancing aid has a removable sterile protection (7) for the lancet which comprises a latching element (40) which prevents a relative rotational movement of the bearing element (5) and the supporting surface (20) as long as the sterile protection (7) is not detached from the lancet.

13. Lancing aid according to claim 12,
in which the sterile protection (7) comprises a barbed hook which prevents a movement of the lancet holder (6) parallel to the puncture direction of the lancet.

14. Lancing aid according to claim 1,
in which the housing has a stop (30) which stops the falling movement of the lancet holder (6).

15. Lancing aid according to claim 14,
in which the falling movement of the lancet holder (6) is stopped by an interaction of the stop (30) and at least one bearing element (5).

16. Lancing aid according to claim 1,
in which the opening of the housing (9) is located in a cap (10) which is movably mounted on the housing (9).

17. Lancing aid according to one of the claims 1 to 16, **characterized in that** it does not have a device for tensioning the spring element.

18. Lancing aid according to one of the claims 1 to 17, **characterized in that** it has a mechanism which actively blocks the lancet after its use.

## Revendications

1. Accessoire perforant comprenant
- un boîtier (9) avec une ouverture dans laquelle un porte-lancette (6) avec une lancette est monté de manière mobile,
- le porte-lancette (6) étant relié à un élément à ressort et le porte-lancette (6) contenant au moins un élément de palier (5), et
- au moins une surface d'appui (20) étant disposée dans le boîtier (9) en ce sens que l'élément de palier (5) repose dans une première position du porte-lancette (6) sur la surface d'appui (20), et
- une unité de déclenchement (1), par laquelle le porte-lancette (6) est transférée dans une seconde position, une touche de déclenchement (2) de l'unité de déclenchement (1) exécutant un mouvement linéaire et le mouvement linéaire étant converti en un mouvement de rotation relatif de l'élément de palier (5) et de la surface d'appui (20) l'un par rapport à l'autre, de sorte que l'élément de palier (5) tombe dans la seconde position du porte-lancette (6) à partir de la surface d'appui (20), l'élément à ressort tendu avant le mouvement de chute se détendant au moins partiellement et déplaçant le porte-lancette (6) par rapport au boîtier (9), de sorte que la pointe (21) de la lancette sort de l'ouverture du boîtier (9), **caractérisé en ce que** l'unité de déclenchement (1) contient un élément d'entraînement de rotation (3), qui est appuyé par le mouvement linéaire de la touche de déclenchement (2) contre l'élément de palier (5), de sorte que l'élément de palier (5) tombe de la surface d'appui (20).

2. Accessoire perforant selon la revendication 1,
sur lequel l'élément à ressort est relié au porte-lancette (6) en ce sens que l'élément à ressort se trouve dans l'état tendu lorsque l'élément de palier (5) repose sur la surface d'appui (20).

3. Accessoire perforant selon la revendication 1,
sur lequel le mouvement linéaire de la touche de déclenchement (2) s'effectue le long de l'axe longitudinal du boîtier (9).

4. Accessoire perforant selon la revendication 1,
sur lequel l'élément d'entraînement de rotation (3) contient au moins un coulisseau élastique, lequel est déformé par le mouvement de la touche de déclenchement (2).

5. Accessoire perforant selon la revendication 1,
sur lequel l'élément d'entraînement de rotation (3) présente au moins une rampe inclinée, allant à l'intérieur du boîtier.

6. Accessoire perforant selon la revendication 1,
sur lequel l'élément de palier (5) est appuyé par l'élément à ressort ou un autre élément à ressort après la sortie de la lancette de l'ouverture du boîtier (9) contre un côté inférieur de la surface d'appui (20).

7. Accessoire perforant selon la revendication 1,
sur lequel le porte-lancette (6) contient deux éléments de palier (5).

8. Accessoire perforant selon la revendication 1,
sur lequel la surface d'appui (20) présente une cavité dans laquelle l'élément de palier (5) du porte-lancette (6) est fixé.

9. Accessoire perforant selon la revendication 1,
sur lequel l'unité de déclenchement (1) présente un élément de verrouillage (40), par lequel un mouvement de rotation relatif de l'élément de palier (5) et de la surface d'appui (20) est empêché aussi longtemps que la touche de déclenchement (2) n'est pas actionnée.

10. Accessoire perforant selon la revendication 9,
sur lequel l'élément de verrouillage (40) s'engage dans une cavité du porte-lancette (6),

11. Accessoire perforant selon la revendication 1 ou 9,
sur lequel le boîtier (9) présente un élément de verrouillage (40) par lequel un mouvement involontaire et relatif de l'élément de palier (5) et de la surface d'appui (20) est empêché.

12. Accessoire perforant selon la revendication 1,
sur lequel l'accessoire perforant présente une protection stérile (7) amovible pour la lancette qui contient un élément de verrouillage (40), par lequel un mouvement de rotation relatif de l'élément de palier (5) et de la surface d'appui (20) est empêché aussi longtemps que la protection stérile (7) n'est pas détachée de la lancette.

13. Accessoire perforant selon la revendication 12,
sur lequel la protection stérile (7) contient un crochet qui empêche un mouvement du porte-lancette (6) parallèlement au sens de perforation de la lancette.

14. Accessoire perforant selon la revendication 1,
sur lequel le boîtier présente une butée (30) par laquelle le mouvement de chute du porte-lancette (6) est stoppé.

15. Accessoire perforant selon la revendication 15,
sur lequel le mouvement de chute du porte-lancette (6) est arrêté par l'action conjuguée de la butée (30) et d'au moins un élément de palier (5).

16. Accessoire perforant selon la revendication 1,
sur lequel l'ouverture du boîtier (9) se trouve dans un capuchon (10) qui est monté de façon mobile sur le boîtier (9).

17. Accessoire perforant selon l'une quelconque des revendications 1 à 17, **caractérisé en ce qu'**il ne présente pas un dispositif pour la tension de l'élément à ressort.

18. Accessoire perforant selon l'une quelconque des revendications 1 à 18, **caractérisé en ce qu'**il présente un mécanisme qui bloque activement la lancette après son utilisation.
